# EUROPÄISCHE PATENTSCHRIFT

(11) **EP 0 401 533 B1**
(45) Veröffentlichungstag und Bekanntmachung des Hinweises auf die Patenterteilung: **30.06.1993**
(21) Anmeldenummer: 90108762.7
(22) Anmeldetag: 10.05.1990
(51) Int. Cl.: C07C 59/153, C07C 51/00

(54) **Verfahren zur kontinuierlichen Herstellung von Glyoxylsäure**
Continuous process for the preparation of glyoxylic acid
Procédé continu pour la préparation de l'acide glyoxylique

(30) Priorität: 05.06.1989 AT 1357/89
(43) Veröffentlichungstag der Anmeldung: 12.12.1990
(73) Patentinhaber: Chemie Linz Gesellschaft m.b.H., A-4021 Linz (AT)
(72) Erfinder: Sajtos, Alexander, Dipl.-Ing., A-4020 Linz (AT); Kloimstein, Engelbert, Ing., A-4070 Eferding (AT); Höllinger, Karl, A-4030 Linz (AT); Farnleitner, Lorenz, Dr., A-4020 Linz (AT)
(74) Vertreter: Kunz, Ekkehard, Dr.

(56) Entgegenhaltungen:
- EP-A- 0 099 981

## Beschreibung

Die Erfindung betrifft ein Verfahren zur kontinuierlichen Herstellung einer reinen konzentrierten wäßrigen Lösung von Glyoxylsäure mit niedriger Farbzahl durch Hydrolyse von Glyoxylsäurealkylesterhemiacetalen.

Glyoxylsäure in Form einer etwa 50 %igen Lösung ist ein wertvolles Ausgangsprodukt für die Herstellung von Pflanzenschutzmitteln, Arzneimitteln, Geruchs- und Aromastoffen, sowie Kunststoffvorprodukten. Insbesondere bei der Verwendung zur Herstellung von Arzneimitteln, Geruchs- und Aromastoffen, sowie Kunststoffvorprodukten werden hohe Anforderungen an die Reinheit, Farbe und Stabilität der Glyoxylsäurelösung gestellt.

Aus der EP-B 0 099 981 ist die Herstellung von Glyoxylsäure durch Ozonolyse von Maleinsäuredialkylestern in alkoholischer Lösung, katalytische Reduktion des entstandenen Peroxids zum Hemiacetal und anschließende Hydrolyse durch Erhitzen mit Wasser beschrieben. Nachteilig dabei ist, daß das bei der Herstellung des Hemiacetals als Nebenprodukt anfallende Dialkyloxalat zu Oxalsäure hydrolisiert wird und etwa 2 - 5 % Oxalsäure als bei der Weiterverarbeitung störende Nebenprodukt in der Glyoxalsäure zurückbleiben. Das bei der Hydrolyse entstehende Alkohol-Wassergemisch wird aus dem Reaktionsgemisch abdestilliert, was bei vollständiger Abtrennung des Alkohols eine relativ hohe thermisch Belastung der Glyoxylsäure und relativ lange Reaktionszeiten mit sich bringt. Diese Belastung der thermisch instabilen Glyoxylsäure führt zur Bildung teilweise unlöslicher, teilweise tiefgefärbter Nebenprodukte, die nicht mehr vollständig abtrennbar sind, wodurch sich durch die höhere Farbzahl Beschränkungen bei der Vewendung so hergestellter Glyoxylsäure ergeben. Zwar ist es möglich die Hydrolyse des Hemiacetals durch Zusatz von Mineralsäure zu beschleunigen, wodurch die Reaktionszeit verkürzt wird, doch bleiben Spuren an Säure als nicht abtrennbare Verunreinigungen im Endprodukt zurück.

Es konnte nun überraschenderweise ein Verfahren zur Herstellung einer reinen konzentrierten wäßrigen Lösung von Glyoxylsäure mit niedriger Farbzahl durch Hydrolyse von Glyoxylsäureesterhemiacetalen gefunden werden, bei dem etwa 50 %ige Glyoxylsäurelösung mit hoher Reinheit, niedriger Farbzahl und guter Farbstabilität in guter Ausbeute unter schonenden Bedingungen gewonnen wird.

Gegenstand der Erfindung ist demnach ein Verfahren zur kontinuierlichen Herstellung einer reinen konzentrierten wäßrigen Lösung von Glyoxylsäure mit niedriger Farbzahl durch Hydrolyse von Glyoxylsäureesterhemiacetalen, das dadurch gekennzeichnet ist, daß Glyoxylsäureesterhemiacetale in einer Reaktorkaskade mit Wasserdampf im Gegenstrom behandelt werden.

Die Ausgangsverbindungen Glyoxylsäureesterhemiacetale können gemäß der bereits erwähnten EP-B-0 099 981 hergestellt werden. Bevorzugt werden solche Glyoxylsäureesterhemiacetale eingesetzt, die bei der Hydrolyse einen Alkohol abspalten, dessen Siedepunkt oder dessen Siedepunkt im Azeotrop mit Wasser unter 100°C liegt. Beispiele für solche Glyoxylsäureesterhemiacetale sind Glyoxylsäuremethylestermethylhemiacetale, Glyoxylsäureethylesterhemiacetale, Glyoxylsäurepropylesterhemiacetal, Glyoxylsäure-i-propylesterhemiacetale, Glyoxylsäure-t-butylesterhemiacetale, Glyoxylsäure-n-butylesterhemiacetale. Besonders bevorzugt wird Glyoxylsäuremethylestermethylhemiacetal eingesetzt.

Zur Durchführung der Reaktion wird das Glyoxylsäurealkylesterhemiacetal gegebenenfalls in Lösung mit einem aliphatischen Alkohol mit 1 bis 4 C-Atomen im oberen Teil der Reaktionskaskade eingebracht und mit Wasserdampf im Gegenstrom behandelt, wobei das Mengenverhältnis Glyoxylsäurealkylesterhemiacetal zu Wasserdampf mindestens 1: 1 bis 1,5 vorzugsweise 1: 1,2 bis 1,35 beträgt, um auch die Hydrolyse gegebenenfalls vorhandener Acetale zu gewährleisten.

Die Reaktorkaskade wird vorzugsweise durch eine Bodenkolonne realisiert, wobei vorteilhafterweise eine Kolonne mit definierter Flüssigkeitsmenge auf den Böden (hold up) verwendet wird, wodurch ein Trockenlaufen der Böden vermieden werden soll.

Die Verweilzeit in der Reaktorkaskade soll eine möglichst quantitative Hydrolyse des Glyoxylalkylesterhemiacetals ermöglichen, aber auch möglichst kurz sein, um die thermische Belastung der Glyoxylsäure gering zu halten. Die dazu notwendige Verweilzeit ist abhängig von der Flüssigkeitsmenge auf den Böden und von der Bodenzahl der Reaktorkaskade. In der Regel sind Verweilzeiten von 30-60 min ausreichend.

Die Kopftemperatur der Reaktorkaskade wird so geregelt, daß der bei der Hydrolyse entstehende Alkohol gemeinsam mit dem überschüssigen Wasserdampf aus der Kolonne ausgetrieben wird. Die Sumpftemperatur der Kolonne wird so geregelt, daß am Boden der Kolonne eine etwa 44 - 46 %ige Lösung von Glyoxylsäure anfällt. In der Regel beträgt die Sumpftemperatur etwa 110 - 125°C, die Kopftemperatur etwa 90 - 100°C, wobei vorzugsweise eine Temperaturdifferenz von mindestens 20°C eingehalten wird.
Die Temperatur am Kopf der Kolonne wird im gesamten Bereich so geregelt, daß ein ausreichender Rücklauf gewährleistet ist. Gleichzeitig mit dem über Kopf abgehenden Alkohol-Wassergemisch werden auch im Ausgangsprodukt gegebenenfalls enthaltene höhersiedende schwer hydrolysierbare und wasserdampfflüchtige Nebenprodukte, wie Dialkyloxalate ausgetrieben, wodurch eine zusätzliche Reinigung erfolgt. Der Gehalt an Oxalsäure im Endprodukt wird dadurch auf etwa 0,5 % reduziert.
Aus dem Sumpf der Kolonne wird eine etwa 44 - 46 %ige Glyoxylsäurelösung abgezogen und anschließend in einen evakuierten Behälter entspannt, wobei gleichzeitig neben der raschen Abkühlung die Lösung auf eine Konzentration von mindestens 50 % aufkonzentriert wird. Der freigesetzte Wasserdampf wird kondensiert und abgezogen.

Vorteilhafterweise wird ein Teil der im Sumpf abgezogenen Glyoxylsäurelösung wieder in das untere Drittel der Kolonne zurückgeführt, wobei durch die erhöhte Konzentration an Glyoxylsäure im unteren Teil der Kolonne die Hydrolyse des restlichen Glyoxylsäurehemiacetals katalytisch beschleunigt wird. Im Sumpf wird dadurch der Alkoholgehalt in freier oder gebundener Form auf weniger als 1 g/l 50 %ige Glyoxylsäurelösung reduziert.

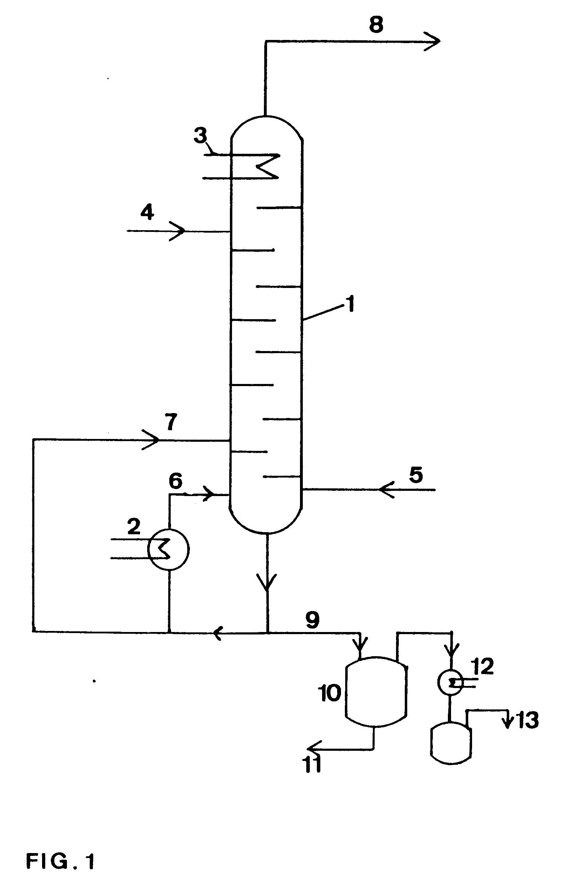

Eine bevorzugte Ausführungsform des erfindungsgemäßen Verfahrens ist in Fig. 1 dargestellt. In Fig. 1 bedeuten 1 eine Reaktorkaskade, vorzugsweise eine Glockenbodenkolonne, 2 einen Erhitzer, 3 einen Dephlegmator, 4 eine Zufuhrleitung des Glyoxylsäurealkylesterhemiacetals, 5 eine Zufuhrleitung des Wasserdampfes, 6 eine Zufuhrleitung der Brüden aus dem Erhitzer 2, 7 eine Zufuhrleitung der Glyoxylsäure für die Rückführung, 8 ein Brüdenrohr, 9 eine Entnahmeleitung für die Glyoxylsäurelösung, 10 einen Vorlagebehälter, 11 eine Entnahmeleitung der gekühlten und konzentrierten Glyoxylsäurelösung, 12 einen Kondensator, 13 eine Vakuumleitung.

Glyoxylsäurealkylesterhemiacetal wird gegebenenfalls in alkoholischer Lösung über Leitung 4 bei einer Tempereratur von etwa 20 - 100 °C in die Kolonne eingebracht.Über Leitung 5 wird überhitzter Wasserdampf eingebracht und durch die Kolonne nach oben geführt.

Im Sumpf der Kolonne wird ein Teil der entstandenen Glyoxylsäurelösung abgezogen, über einen Erhitzer 2 erhitzt und über Leitung 6 die Brüden in die Kolonne zurückgeführt. Ein Teil der im Sumpf abgezogenen Glyoxylsäure wird über Leitung 7 in das untere Drittel der Kolonne zurückgeführt. Über Leitung 9 wird etwa 44 - 46 %ige Glyoxylsäurelösung einer Temperatur von 110 - 125°C abgezogen in einen evakuierten Vorlagebehälter 10 bei etwa 50 mbar entspannt, gekühlt, wobei im Kondensator 12 der entweichende Wasserdampf kondensiert wird. Am Kopf der Kolonne wird die Temperatur durch einen Dephlegmator 3 so geregelt, daß durch das Brüdenrohr 8 das Alkohol-Wassergemisch, sowie gegebenenfalls vorhandene höhersiedende, schwer hydrolysierbare und wasserdampfflüchtige Nebenprodukte, entweichen.

Nach dem beschriebenen Verfahren wird Glyoxylsäure in Ausbeuten von 95 - 98 % bezogen auf das Ausgangsprodukt in hoher Reinheit erhalten. Die mittlere Verweilzeit beträgt im allgemeinen nur zwischen 30 und 60 min, wodurch die Bildung unlöslicher oder nicht abtrennbarer Nebenprodukte, die die Reinheit und Farbe der Glyoxylsäure beeinträchtigen, vermieden wird.

Die erfindungsgemäß hergestellte Glyoxylsäure weist eine Gardener Farbzahl (ASTM D 1544-8) von 1 bis 2 auf, die sich auch bei längerer Lagerung nicht verschlechtert.

### Beispiel 1:

In einer Versuchsapparatur lt. Fig. 1, bestehend aus einer Glockenbodenkolonne mit 27 Böden und 300 mm Durchmesser wurden stündlich an der Stelle 4 am 21. Boden 85 kg Glyoxylsäuremethylesterhemiacetal und an der Stelle 5 115 kg Wasserdampf mit einem Vordruck von 7 bar eingebracht. Gleichzeitig wurden an der Stelle 7 am 7. Boden ca. 30 kg heißes Sumpfprodukt zurückgeführt. Der Wärmetauscher 2 wurde mit 3,3 bar Dampf beheizt. Es stellte sich eine Sumpftemperatur von 114 - 115°C ein. Die Kopftemperatur unter dem Dephlegmator lag zwischen 96 und 98°C. Unter diesen Bedingungen wurden stündlich 113 kg Glyoxylsäurelösung als Sumpf der Kolonne in den Behälter 10 ausgetragen. Durch die Vakuumverdampfung bei ca. 65 mbar wurden stündlich noch 10 - 12 l Wasser verdampft und etwa 100 kg 51 bis 52 %ige praktisch farblose Glyoxylsäurelösung erhalten. Das Produkt enthielt weniger als 1 g Methanol/kg. Bei einem Gesamtholdup der Kolonne von 60 l ergab das eine durchschnittliche Verweilzeit der GS im Reaktionssystem von 40 bis 45 Minuten.

Die erhaltene Glyoxylsäurelösung wies eine Gardener Farbzahl von 1 - 2 auf und hatte folgende Zusammensetzung
- Glyoxylsäure: 51,9 %
- Glyoxal: nicht nachweisbar
- Oxalsäure: 0,5 %
- Bernsteinsäure: nicht nachweisbar
- Maleinsäure: nicht mehr nachweisbar
- Methanol: 70 ppm
- Ameisensäure: nicht nachweisbar

### Vergleichsbeispiel

In einen 1500 l Reaktionskessel mit Rührwerk, Dampfheizung und einer 3 m langen Füllkörperkolonne und Kondensator mit Rücklaufteiler wurden 500 kg Glyoxylsäuremethylesterhemiacetal und 600 kg Wasser vorgelegt und unter Rühren zum Sieden erhitzt. Sobald Rückflußkochen einsetzte, wurde mit der Destillatabnahme begonnen. Bei einer Kopftemperatur von etwa 75°C wurde das abgespaltene Methanol im Lauf von 5 Stunden zu mehr als 90 % abdestilliert. Nun wurde die Kopftemperatur bis auf 97 °C gesteigert, dabei sank der methanolgehalt im Verlauf von weiteren 3 Stunden auf weniger als 5 g/kg Reaktionslösung ab. Bei einer Siedetemperatur von 108 - 110°C wurden 616 kg hellbraun gefärbte 50 %ige Glyoxylsäurelösung erhalten. Weiters erhielt man 484 kg Methanol-Wassergemisch als Destillat, aus dem Methanol zurückgewonnen werden kann.

Die so hergestellte Glyoxylsäure wies eine Gardener Farbzahl von 7 - 8 auf und war für die Verwendung als Ausgangsverbindung für die Herstellung von Pharmazeutika, Geruchs- und Aromastoffen, sowie Kunststoffvorprodukten nicht geeignet.

Zusammensetzung
- Glyoxylsäure: 50 %
- Glyoxal: nicht nachweisbar
- Oxalsäure: 4 %
- Bernsteinsäure: nicht nachweisbar
- Maleinsäure: nicht nachweisbar
- Methanol: 5 g/kg
- Ameisensäure: nicht nachweisbar

## Patentansprüche

1. Verfahren zur kontinuierlichen Herstellung einer reinen konzentrierten wäßrigen Lösung von Glyoxylsäure mit niedriger Farbzahl durch Hydrolyse von Glyoxylsäureesterhemiacetalen, dadurch gekennzeichnet, daß Glyoxylsäureesterhemiacetale in einer Reaktorkaskade mit Wasserdampf im Gegenstrom behandelt werden.

2. Verfahren nach Anspruch 1, dadurch gekennzeichnet, daß Glyoxylsäureesterhemiacetale eingesetzt werden, die bei der Hydrolyse Alkohole freisetzen, deren Siedepunkt oder deren Siedepunkt im Azeotrop mit Wasser unter 100°C liegt.

3. Verfahren nach Anspruch 2, dadurch gekennzeichnet, daß Glyoxylsäuremethylestermethylhemiacetal eingesetzt wird.

4. Verfahren nach einem der Ansprüche 1 bis 3, dadurch gekennzeichnet, daß das Mengenverhältnis Glyoxylsäureesterhemiacetal : Wasserdampf 1: 1,2 bis 1,35 beträgt.

5. Verfahren nach einem der Ansprüche 1 bis 4, dadurch gekennzeichnet, daß die Verweilzeit in der Reaktorkaskade 30 - 60 min beträgt.

6. Verfahren nach einem der Ansprüche 1 bis 5, dadurch gekennzeichnet, daß ein Teil der am untersten Boden der Kaskade abgezogenen Glyoxylsäurelösung wieder in die Reaktorkaskade zurückgeführt wird.

7. Verfahren nach einem der Ansprüche 1 bis 6, dadurch gekennzeichnet, daß die am untersten Boden abgezogene Glyoxylsäurelösung anschließend im Vakuum aufkonzentriert wird.

8. Verfahren nach einem der Ansprüche 1 bis 7, dadurch gekennzeichnet, daß im Sumpf der Reaktorkaskade eine Temperatur von 110 - 125°C und am Kopf der Reaktorkaskade eine Temperatur von 90 - 100°C aufrecht erhalten wird.

9. Verfahren nach einem der Ansprüche 1 - 8, dadurch gekennzeichnet, daß zwischen Sumpf und Kopf der Reaktorkaskade eine Temperaturdifferenz von mindestens 20°C aufrecht erhalten wird.

## Claims

1. Process for the continuous preparation of a pure concentrated aqueous solution of glyoxylic acid having a low colour number by hydrolysis of glyoxylic acid ester hemiacetals, characterized in that glyoxylic acid ester hemiacetals are treated with steam in counter-current in a reactor cascade.

2. Process according to Claim 1, characterized in that glyoxylic acid ester hemiacetals are used which, on hydrolysis, release alcohols whose boiling point, or whose point in the azeotropic with water, is below 100°C.

3. Process according to Claim 2, characterized in that methyl glyoxylate methyl-hemiacetal is used.

4. Process according to one of Claims 1 to 3, characterized in that the glyoxylic acid ester hemiacetal: steam mass ratio is 1:1.2 to 1.35.

5. Process according to one of Claims 1 to 4, characterized in that the residence time in the reactor cascade is 30 - 60 minutes.

6. Process according to one of Claims 1 to 5, characterized in that a part of the glyoxylic acid solution taken off at the lowest tray of the cascade is recycled into the reactor cascade.

7. Process according to one of Claims 1 to 6, characterized in that the glyoxylic acid solution taken off at the lowest tray is then concentrated in vacuo.

8. Process according to one of Claims 1 to 7, characterized in that a temperature of 110 - 125°C is maintained in the bottom of the reactor cascade and a temperature of 90 - 100°C is maintained at the top of the reactor cascade.

9. Process according to one of Claims 1 - 8, characterized in that a temperature difference of at least 20°C is maintained between the bottom and top of the reactor cascade.

## Revendications

1. Procédé pour la préparation en continu d'une solution aqueuse pure concentrée d'acide glyoxylique, avec un faible indice de coloration, par hydrolyse des hémiacétals d'esters d'acide glyoxylique, caractérisé en ce que l'on traite les hémiacétals d'esters d'acide glyoxylique dans une cascade de réacteurs avec de la vapeur d'eau à contre-courant.

2. Procédé selon la revendication 1, caractérisé en ce que l'on traite les hémiacétals d'esters d'acide glyoxylique qui libèrent des alcools par hydrolyse dont le point d'ébullition ou le point d'ébullition de l'azéotrope avec l'eau est inférieur à 100°C.

3. Procédé selon la revendication 2, caractérisé en ce que l'on utilise le méthylique hemiacétal de l'ester méthylique de l'acide glyoxylique.

4. Procédé selon l'une des revendications 1 à 3, caractérisé en ce que le rapport de masse entre l'hémiacétal de l'ester de l'acide glyoxylique et la vapeur d'eau est de 1:1,2 à 1,35.

5. Procédé selon l'une des revendications 1 à 4, caractérisé en ce que le temps de réaction est de 30 à 60 minutes dans la cascade de réacteurs.

6. Procédé selon l'une des revendications 1 à 5, caractérisé en ce qu'une fraction de la solution d'acide glyoxylique prélevée à la partie inférieure de la cascade est réintroduite dans la cascade de réacteur.

7. Procédé selon l'une des revendications 1 à 6, caractérisé en ce que la solution d'acide glyoxylique prélevée à la partie inférieure est immédiatement après concentrée sous vide.

8. Procédé selon l'une des revendications 1 à 7, caractérisé en ce que l'on maintient une température de 110 à 125°C en queue de colonne et une température de 90 à 100°C en tête de colonne.

9. Procédé selon l'une des revendications 1 à 8, caractérisé en ce que l'on maintient une différence de température d'au moins 20°C entre la tête et la queue de la colonne.
